# EUROPÄISCHE PATENTSCHRIFT

(11) **EP 2 299 954 B1**
(45) Veröffentlichungstag und Bekanntmachung des Hinweises auf die Patenterteilung: **30.09.2015**
(21) Anmeldenummer: 09765596.3
(22) Anmeldetag: 16.06.2009
(51) Int. Cl.: A61F 13/14

(54) **VERFAHREN ZUM SCHUTZ VON HAUTBEREICHEN EINER ZITZE WÄHREND DES MELKVORGANGES, SOWIE FILM FÜR EIN SOLCHES VERFAHREN**
METHOD FOR THE PROTECTION OF SKIN REGIONS OF A TEAT DURING THE MILKING PROCESS, AND FILM FOR SUCH A METHOD
PROCÉDÉ DE PROTECTION DE ZONES CUTANÉES D'UN TRAYON PENDANT LA TRAITE ET FILM POUR UN TEL PROCÉDÉ

(30) Priorität: 19.06.2008 DE 102008029172; 16.12.2008 DE 202008017000 U
(43) Veröffentlichungstag der Anmeldung: 30.03.2011
(73) Patentinhaber: Kenndoff, Jochen, 21075 Hamburg (DE)
(72) Erfinder: Kenndoff, Jochen, 21075 Hamburg (DE)
(74) Vertreter: Emmel, Thomas
(86) Internationale Anmeldenummer: PCT/EP2009/004311
(87) Internationale Veröffentlichungsnummer: WO 2009/153016

(56) Entgegenhaltungen:
- EP-A- 1 695 721
- WO-A-94/07935
- US-A- 5 714 225

## Beschreibung

Die Erfindung bezieht sich auf ein Verfahren, mit dem Hautbereiche von Zitzen mit einem Film während und zwischen den Melkvorgängen geschützt werden können. Weiterhin bezieht sich die Erfindung auf Filme, die in dem Verfahren zur Anwendung kommen können.

Die wirksame Betreuung und Haltung von großen Milchtierherden zur Erzeugung von Molkereiprodukten ist eine der Hauptaufgaben in der Landwirtschaft. Erkrankungen sowie starke Abweichungen von der Herdennorm einzelner Tiere werden so zu einem ernst zu nehmenden Problem, da die Wirtschaftlichkeit heute größtenteils über ein effizientes, reibungsloses Management sehr großer Herden erreicht wird. Erkrankungen einzelner Tiere sowie Abweichungen von der Herdennorm speziell im Bereich der Zitze stellen einen erheblichen Störfaktor dar, der die Wirtschaftlichkeit, insbesondere beim Einsatz von halbautomatischen und vollautomatischen Melkrobotern, beeinträchtigt.

Grundsätzlich gilt, dass die Zitzen durch den Melkvorgang stark beansprucht werden, was zu Reizungen bzw. dem Bruch insbesondere bereits gereizter Haut führen kann. Selbst durch solche kleinen und kleinsten Verletzungen kann durch mikrobiellen Angriff eine Mastitis ausgelöst werden.

Ist die Haut der Zitze äußerlich verletzt, müssen die Tiere i.d.R. getrennt gehalten und mit einem deutlichen Mehraufwand versorgt werden.

Schon kleinste Verletzungen im Bereich der Zitze, die Sekret absondern können, führen zwangsläufig zu einer Absonderung des betroffenen Tieres, da andernfalls die Milch mit Blut oder Wundsekret verunreinigt werden kann, was aufgrund der nachgeschalteten Qualitätskontrolle insbesondere moderner automatisierter Qualitätskontrollsysteme zum Verwerfen des Ermolkenen i.d.R. aus allen gemolkenen Vierteln führt.

Bei etwas gröberen Verletzungen muss das der Zitze zugehörige Euterviertel u.U. sogar trockengestellt werden, um eine Heilung zu ermöglichen, da die Zitze durch den Melkvorgang ganz erheblich belastet wird. Gebildeter Schorf wird durch die hohe Druck- und Zugbelastung i.d.R. wieder aufgerissen. Im ungünstigsten Fall führt dies sogar zu einer Vergrößerung einer ursprünglich kleinen Verletzung an der Zitze.

Es sind bislang keine in großen Milchtierherden mit vertretbarem Aufwand einsetzbare Schutzmöglichtkeiten der Zitze während des Melkvorgangs bekannt.

Was bekannt ist, sind Möglichkeiten, eine bereits verletzte Zitze zu versorgen, wobei dann aber in aller Regel keine oder nur noch eine erschwerte Melkfähigkeit der versorgten Zitze gegeben ist.

Üblich bei Verletzungen der Haut an der Zitze ist das Anlegen eines Zitzenverbandes. Dies kann z.B. durch Tauchbäder, durch nicht auf der Haut haftende, aber auf sich selbst haftende Verbände oder aber auch durch flächige, selbstklebende Pflastermaterialien, die grundsätzlich zur Abdeckung von Wunden geeignet sind, erfolgen.

Alle diese heute gängigen Lösungen zum Schutz und zur Versorgung der Zitze haben verschiedene Nachteile.

Tauchbäder, die z.B. natürlichen Kautschuk enthalten, können sensibilisierend wirken. Verbände, die mit Hilfe von Tauchbädern aufgebracht werden und i.d.R. sicher schützen, müssen z.B. vor jedem Melkvorgang wieder entfernt werden, um den Melkvorgang überhaupt zu ermöglichen. Der Aufwand für das Entfernen des Verbandes ist zum Teil erheblich. So müssen die Verbände z.B. eingeweicht werden, bevor sie wieder entfernt werden können. Im schlimmsten Fall entstehen durch die Abnahme des Verbandes selbst Reibe- und Quetschverletzungen. Wie aufwändig das Prozedere ist, ergibt sich z.B. aus der Patentschrift DE 699 14 751 für eine polyurethanbasierte Tauchlösung. Darin werden auch die Nachteile bezüglich der Haltbarkeit von wasserlöslichen Filmen beschrieben.

Zitzenverbände mit flächigen, selbstklebenden Pflastermaterialien werden hauptsächlich zur Fixierung eingesetzt. Fixomull (BSN) wird z.B. hierzu als elastischer Fixierverband häufig genutzt. Der Verband ist nicht dafür konzipiert, in direkten Kontakt mit der Wunde gebracht zu werden, sondern wird im Wesentlichen zur Fixierung z.B. von Zitzenstiften angelegt und muss zum Melken wieder abgenommen werden. Als dauerhafter Schutz, der auf der Zitze auch während des Melkens verbleibt, ist ein derartiger Zitzenverband daher ungeeignet. Die hier z.B. eingesetzten Acrylatklebeschichten kleben bei der hohen Haftkraft so, dass sie die Haut bei Belastung reizen. Da die Klebeschicht in einer nur sehr dünnen Schicht auf dem flexiblen Träger aufgebracht ist, kann die Klebeschicht Ein erfindungsgemässer Film ist ein auf sich selbst haftend ausgebildeter Zuschnitt aus einem Verbundmaterial aus einer Klebeschicht mit einem einseitig darauf angeordneten flächigen Träger. Die Klebeschicht weist in einer ersten Variante eine Dicke und plastische und/oder elastische Verformbarkeit auf, die gewährleistet, dass die Klebeschicht bei Dehnung der Zitze während des Melkvorgangs von dem Film auf die Haut wirkende Scherkräfte kompensiert.

Alternativ oder ergänzend ist die Klebeschicht in einer zweiten Variante so ausgebildet, dass sie die sich bei einer in Längsrichtung der Zitze auf sie wirkenden Kraft von der Haut ohne wesentliche Verletzung der Corneozytenschieht ablöst.

Bevorzugt werden bei der ersten Variante des erfindungsgemässen Films Dicke und Verformbarkeit der Klebeschicht so gewählt, dass sie bei Dehnung der Zitze während des Melkvorganges nicht oder nur geringfügig von der Haut gelöst wird. Dies ist möglich, da ein Großteil der durch das Melkzeug auf den Film übertragenen Scherkräfte nicht auf die Haut übertragen, sondern in der Klebeschicht durch Verformung derselben egalisiert werden, bevor sie auf die Haut wirken können.

Erfindungsgemäss beträgt die Dicke der eingesetzten Klebeschicht 150 - 3000 µm, bevorzugt 200 - 2000 µm, besonders bevorzugt 250 - 1000 µm. Die Verformbarkeit der Klebeschicht ist so beschaffen, dass sich der Träger eines auf Stahl flächig verklebten Filmes um mindestens 100% seiner Höhe bevorzugt mehr 300 % besonders bevorzugt mehr als 500 % vom Rand des Pflasters zur Mitte des Pflaster hin zurückschieben lässt, ohne dass sich die Klebeschicht von der Stahlplatte löst. Mit anderen Worten der Träger lässt sich bei eikann das Tier in der Herde nicht mehr zur Milchproduktion herangezogen werden und muss abgegeben oder geschlachtet werden.

Es besteht daher ein Bedürfnis, die Zitzen während des Melkvorgangs zu schützen, bzw. Abweichungen bei nicht Herdennorm-gerechten Zitzen zu kompensieren.

Gelöst wird diese Aufgabe mit einem Verfahren gemäss Anspruch 1 sowie einem in dem Verfahren einsetzbaren Film gemäss Anspruch 5.

Erfindungsgemäß ist vorgesehen, dass insbesondere verletzte oder gestresste Hautbereiche einer Zitze speziell während des Melkvorganges insbesondere überlappend mit einem auf sich selbst haftenden und über eine elastische Klebeschicht auf der Haut haftenden Film umwickelt wird, welcher für die Dauer mehrerer Tage auch zwischen den wiederkehrenden Melkvorgängen zum Schutz auf der Zitze verbleiben kann. Weiterhin kann man über die Dicke der Wicklung falls erforderlich den Durchmesser einer nicht Herdennorm-gerechten Zitze an ein Melkgeschirr anpassen.

Mit Film soll im Folgenden ein Zuschnitt aus einem Verbundmaterial aus einem flächigen Träger und der Klebeschicht bezeichnet werden. Über die Klebschicht haftet der Film auf sich selbst und an der Haut.

Grundsätzlich geeignete Filme sind dem Fachmann aus z.B. dem Gebiet der Pflaster- oder Verbandmaterialien bekannt. Grundsätzlich können unter Berücksichtigung der nachfolgenden Ausführungen alle in diesem Zusammenhang bekannten Träger- bzw. Klebematerialien im Rahmen der Erfindung zum Einsatz kommen.

In einer ersten Variante des erfindungsgemässen Verfahrens ist die Klebeschicht mit einer Dicke und Verformbarkeit gewählt, derart, dass sie bei Dehnung der Zitze während des Melkvorgangs von dem mit dem Melkgeschirr in Kontakt kommenden Träger auf die Haut wirkende Scherkräfte kompensiert.

Alternativ oder ergänzend kann die Klebeschicht in einer zweiten Variante des erfindungsgemässen Verfahrens so gewählt sein, dass sie sich bei einer in Längsrichtung der Zitze auf sie wirkenden Kraft von der Haut ohne wesentliche Verletzung der Corneozytenschicht ablöst.

Es hat sich überraschend herausgestellt, dass das erfindungsgemässe Verfahren, nicht Herdennorm-gerechte, gestresste oder verletzte Zitzen während des Melkvorgangs effektiv schützt. Das Verfahren lässt sich nicht nur auf verletzten bzw. gereizten Zitzen anwenden, sondern kann selbstverständlich auch auf intakten nicht gestressten oder gereizten Zitzen angewendet werden. In allen Fällen kann der zum Schutz aufgebrachte Film problemlos über mehrere Tage und Melkintervalle an der Zitze verbleiben.

In einer Variante ist der in dem erfindungsgemässen Verfahren eingesetzte Film mit einer Klebeschicht vorkonfektioniert, die eine Dicke und Verformbarkeit aufweist, die gewährleistet, dass die Klebeschicht bei Dehnung der Zitze während des Melkvorgangs von dem Melkzeug auf den Film wirkende Scherkräfte kompensiert, sodass diese die Haut nicht zusätzlich belasten.

Bevorzugt ist die Klebeschicht dergestalt elastisch ausgebildet, dass sie sich nach Wegfall der auf sie wirkenden Scherkräfte im wesentlichen wieder auf ihre Ausgangsform, d.h. die Form, die sie vor Einwirkung der Scherkräfte hatte, rückverformt.

Bevorzugt ist die Klebeschicht bei dieser Variante weiterhin so gewählt, dass sie sich während des Melkvorgangs nicht von der Haut ablöst. Bei dieser Ausgestaltung wird die Haut besonders geschont.

Alternativ oder in Kombination mit den oben genannten Eigenschaften kann die in dem Film vorgesehene Klebeschicht so gewählt sein, dass sie sich bei Streckung des Films bzw. Trägers in Richtung der Zitzendehnung inkrementell und entgegengesetzt zur Streckrichtung von der Haut löst und anschließend wieder mit der Haut verklebt. Erfindungswesentlich ist, dass sich die Klebschicht ohne wesentliche Verletzung der Corneozytenschicht von der Haut löst.

Klebschichten mit solchen Eigenschaften sind im Bereich von Wundpflastern bekannt. Sie erlauben eine schmerzfreie Entfernung von Pflastermaterialien von der Haut. Aus dem US-Patent 4224313 ist z.B. ein entsprechender selbsthaftender, dehnbarer und flexibler Verband bekannt, der durch Längsstreckung parallel zu Verklebungsfläche einfach und weitgehend schmerzfrei von der Hautoberfläche gelöst werden kann. Durch Streckung wird am ersten Kontaktpunkt die Haftkraft zwischen Klebeschicht und Oberfläche die Haftkraft durch die Zugkraft überschritten. In der Regel löst sich die Klebeschicht von der Haut ohne Corneozyten abzunehmen.

Bei beiden Varianten sind die Klebepunkte dicht an dicht auf der Haut der ungedehnten Zitze und überspannen auch i.d.R. vorhandene Faltungen der Haut und es ist gewährleistet, dass die durch den Film abgedeckte Haut während des Melkvorganges nicht zusätzlich durch die klebende Verbindung gereizt wird. Im ersten Fall macht der Klebstoff die Bewegung der Haut mit, d.h. die Verbindungen zwischen Haut und Klebeschicht in den einzelnen Klebepunkten bleiben erhalten.

Im zweiten Fall löst sich der Klebstoff inkrementell von der Haut ab, sodass die Corneozytenschicht nicht oder nur wenig beschädigt wird und egalisiert über das Lösen und Wiederverkleben auftretende Spannungen automatisch. In diesem Fall ist es zwar vorteilhaft aber nicht zwingend erforderlich, dass die Klebeschicht die einwirkenden Zugkräfte kompensiert, da bei beginnender Dehnung die Verklebung sich inkrementell löst, sodass sich während des Melkprozesses der Film zu großen Teilen lösen kann, aber anschließend sofort wieder spannungsfreier verklebt, wenn der Massagedruck des Melkzeugs auf Verband und Zitze einwirkt.

Vorteilhaft ist die Klebeschicht in einer Ausgestaltung der Erfindung so gewählt, dass sie bis zu einem vorgegebenen Wert Scherkräfte kompensiert und sich oberhalb des Werts weitgehend verletzungsfrei von der Haut ablöst und Unterschreiten des Wertes wieder mit der Haut verklebt. Mit dieser Ausgestaltung, die beide Effekte miteinander kombiniert, kann man auch extreme Bedingungen, z.B. wenn die Zitze stärker gedehnt wird als üblich, bzw. eine nicht optimale Anordnung des Films etc. kompensieren.

Gut geeignet sind grundsätzlich z.B. auch Schäume aus dreidimensional vernetzten Klebemassen, wie oben beschrieben z.B. aus Polyurethan, die kurz vor oder während der stattfindenden Vernetzung physikalisch (z.B. durch Einschlagen von Gas wie z.B. Stickstoff, Kohlendioxid, Edelgasen oder Gemischen hieraus), durch Gasbildung, hervorgerufen durch eine chemische Reaktion (z.B. durch Bildung von Kohlendioxid aus einer Reaktion von Isocyanat mit Wasser) oder durch Verdampfung eines niedrig siedenden Lösungsmittels während der Vernetzung (z.B. Pentan) gebildet werden können. Derartige Schäume sind gleichfalls klebend, kleben subjektiv sanfter und können durch ihre poröse Struktur schneller Sekret aufnehmen verglichen mit ihren ungeschäumten Varianten.

In einer weiteren bevorzugten Ausgestaltung des Verfahrens ist vorgesehen, dass die Zitze mehrfach mit dem Film umwickelt wird. Man erreicht so einen besonders effektiven Schutz gegen von aussen einwirkende traumatische Ereignisse.

Wie erwähnt bezieht sich die Erfindung nicht nur auf ein Verfahren sondern auch auf ein in dem Verfahren einsetzbaren Film zum Schutz von Hautbereichen einer Zitze während des Melkvorgangs.

Wie erwähnt bezieht sich die Erfindung nicht nur auf ein Verfahren sondern auch auf ein in dem Verfahren einsetzbaren Film zum Schutz von Hautbereichen einer Zitze während des Melkvorgangs.

Ein erfindungsgemässer Film ist ein auf sich selbst haftend ausgebildeter Zuschnitt aus einem Verbundmaterial aus einer Klebeschicht mit einem einseitig darauf angeordneten flächigen Träger. Die Klebeschicht weist in einer ersten Variante eine Dicke und plastische und/oder elastische Verformbarkeit auf, die gewährleistet, dass die Klebeschicht bei Dehnung der Zitze während des Melkvorgangs von dem Film auf die Haut wirkende Scherkräfte kompensiert.

Alternativ oder ergänzend ist die Klebeschicht in einer zweiten Variante so ausgebildet, dass sie die sich bei einer in Längsrichtung der Zitze auf sie wirkenden Kraft von der Haut ohne wesentliche Verletzung der Corneozytenschicht ablöst.

Bevorzugt werden bei der ersten Variante des erfindungsgemässen Films Dicke und Verformbarkeit der Klebeschicht so gewählt, dass sie bei Dehnung der Zitze während des Melkvorganges nicht oder nur geringfügig von der Haut gelöst wird. Dies ist möglich, da ein Großteil der durch das Melkzeug auf den Film übertragenen Scherkräfte nicht auf die Haut übertragen, sondern in der Klebeschicht durch Verformung derselben egalisiert werden, bevor sie auf die Haut wirken können.

Erfindungsgemäss beträgt die Dicke der eingesetzten Klebeschicht 150 - 3000 µm, bevorzugt 200 - 2000 µm, besonders bevorzugt 250 - 1000 µm. Die Verformbarkeit der Klebeschicht ist so beschaffen, dass sich der Träger eines auf Stahl flächig verklebten Filmes um mindestens 100% seiner Höhe bevorzugt mehr 300 % besonders bevorzugt mehr als 500 % vom Rand des Pflasters zur Mitte des Pflaster hin zurückschieben lässt, ohne dass sich die Klebeschicht von der Stahlplatte löst. Mit anderen Worten der Träger lässt sich bei einer 1 mm dicken Klebeschicht in dieser Ausgestaltung um mindestens 1 mm, bevorzugt um > 3 mm, besonders bevorzugt um > 5 mm zurückschieben.

Weiterhin bevorzugt ist vorgesehen, dass die Klebeschicht mit einer Elastizität ausgestattet ist, die besonders bevorzugt so eingestellt ist, dass sich nach Freigabe des Trägers bei der oben angegebenen Untersuchungsmethode, die Klebeschicht wieder in ihre ursprüngliche Form rückverformt.

Bei Befestigung des Films mittels einer Klebeschicht liegen die Klebepunkte der Klebeschicht üblicherweise dicht an dicht auf der Haut (entsprechend der Oberflächenstruktur der Haut an den erhabenen Bereichen der Hautoberfläche) der ungedehnten Zitze und überspannen auch i.d.R. tiefer liegende Bereiche oder vorhandene Falten auf der Haut. Bei zu geringer Verformbarkeit bzw. Elastizität, kann die Klebeschicht die einwirkenden Zugkräfte nicht vollständig kompensieren, und in den Zugbereichen wird die Haftkraft der Klebeschicht zur Haut überschritten. Die Folge ist, dass sich während des Melkprozesses der Film zu großen Teilen löst.

Diese Neigung der Klebeschicht sich unter Zugbelastung von der Zitze zu lösen nimmt zu, je dünner die Haftschicht ausgebildet ist. Je dicker die Haftschicht ist, desto mehr der Zugkraft kann in der Klebeschicht durch deren Verformung absorbiert werden.

Dicke, Elastizität und ggf. plastische Verformbarkeit der Klebeschicht sind daher vorteilhaft so aufeinander abgestimmt, dass bei den während des Melkprozesses einwirkenden Kräften die Klebeschicht mindestens die Streckung der Zitze mitmachen kann, ohne sich signifikant von der Zitzenhaut zu lösen,

Besonders vorteilhaft ist weiterhin, wenn nicht nur die Klebeschicht sondern der Film insgesamt so ausgebildet ist, dass er bei üblichen in Melkgeschirren auftretenden Zugkräften um mind. 50% seiner Erstreckung in Richtung der Zitze dehnbar ist. Bevorzugt kann sich der Film entsprechend der auftretenden Längung der Zitze dehnen.

Beide Bestandteile des Films sind erfindungswesentlich. Im folgenden soll insbesondere auf weitere Details der Klebeschicht eingegangen werden, da diese in direkten Kontakt mit der zu schützenden Haut der Zitze kommt. Im wesentlichen gelten die folgenden Ausführungen für beide Varianten der Erfindung.

Eine wesentliche Eigenschaft von selbsthaftenden Klebschichten ist die Fließfähigkeit ihrer Gerüststruktur. Hier kann grob unterschieden werden zwischen Klebeschichten, die in physiologischen Temperaturbereichen und insbesondere unter Druck zu fließen beginnen (sog. kalter Fluß) und Klebeschichten, die dies nicht zeigen.

Klebeschichten die kalten Fluss zeigen, haften i.d.R. einige Zeit nach Applikation auf der Haut deutlich fester, da sie sich der unregelmäßigen Oberflächenstruktur durch das Verfließen anpassen und um feine Härchen herumfließen können und sich damit die Klebefläche, die letztendlich mit der Haut in Kontakt kommt, pro Flächeneinheit deutlich vergrößert.

Gut haftende Klebeschichten mit 1,5 - 4 N/cm Klebkraft auf Stahl, die kalten Fluss zeigen, neigen daher zum sogenannten Corneozytenstripping und reißen in der Regel die jeweils oberste Hautschicht beim Ablösen von der Haut ab.

Klebeschichten, die keinen kalten Fluss zeigen, haften nur durch den Andruck bei und ggf. auch während der Applikation. Bei Klebeschichten ohne kalten Fluss beobachtet man ein Corneozytenstripping auch bei den zuvor genannten Klebkraftwerten i.d.R. nicht. Die Klebeschichten lassen sich auch nach dem Entfernen von der Haut wieder mit dieser verkleben, wobei ggf. die Haftkraft auf der Haut dann etwas reduziert sein kann.

Im Rahmen der Erfindung werden Klebeschichten eingesetzt, die aufgrund ihrer in der Regel weitestgehend kovalent vernetzten Gerüststruktur per se keinen kalten Fluss bzw keinen die erfindungsgemässe Anwendung beeinträchtigenden kalten Fluss zeigen, aber dennoch eine hohe Klebkraft aufweisen. Hierzu zählen Polyurethanklebschichten, z.B. dreidimensional vernetzte Polyurethanklebeschichten, Silikonklebeschichten, Acrylatklebeschichten, synthetische Kautschukklebeschichten sowie Hydrogele. Es handelt sich um eine nicht abschließende Aufzählung einiger Beispiele für geeignete Klebeschichten. Im Rahmen der Erfindung können selbstverständlich auch weitere einem Fachmann bekannte Klebeschichten eingesetzt werden.

Im Rahmen der Erfindung bevorzugte Klebeschichten ohne kalten Fluss weisen Klebkräfte auf Stahl > 0,5 N/cm, bevorzugt, > 0,7 N/cm, besonders bevorzugt >1 N/cm auf. Die angegebenen Klebkräfte beziehen sich auf Klebkraftmessungen auf standardisierten Stahlplatten, da ein Kleben auf der Haut nur unter erheblichem Aufwand direkt gemessen werden kann. Es handelt sich dabei um ein dem Fachmann bekanntes Standardverfahren.

Es ist weiterhin für das erfindungsgemässe Verfahren und auch den Film wichtig, dass die eingesetzten Klebeschichten unter Einwirkung von Druck auf einer hautwarmen Oberfläche ausreichend formstabil sind. Die Klebeschicht sollte sich durch Einwirkung des Massagedrucks des Melkzeuges in Verbindung mit der physiologischen Hauttemperatur auf der Zitze nicht dauerhaft verformen. Wenn sich die Klebeschicht und damit der Film entsprechend der Druckverhältnisse dauerhaft verformt, kann bei wiederholtem Anlegen des Melkzeuges die Effizienz des Melkprozesses über die Zeit beeinträchtigt werden, da die glatte Oberfläche des Melkzeuges beim wiederholten Anlegen nicht mehr auf einer glatten Oberfläche des Films anliegt, sondern auf einer entsprechend den Druckverhältnissen des ersten Melkdurchgangs verformten Oberfläche.

Bevorzugt sind daher solche Klebeschichten, die sich unter Druck nicht dauerhaft, sondern elastisch verformen, so dass sie nach Abnehmen des Melkzeuges ihre ursprüngliche Form wieder nahezu vollständig annehmen.

Weiterhin sollte die erfindungsgemäss eingesetzte Klebeschicht ausreichend wasserdampfdurchlässig sein, damit die darunter liegende Haut insbesondere nach längerer Tragedauer nicht mazeriert. Mazeration ist nicht erwünscht, da hierdurch die die Haut gegenüber dem Angriff von Keimen deutlich anfälliger wird. Nicht wasserdampfdurchlässige oder feuchtigkeitsabsorbierende Klebeschichten können zwar durch Perforation durchlässig gemacht werden. Zeigen sie jedoch kalten Fluss, dann können sich die Poren speziell bei längerer Anwendung und unter Druck leicht wieder schließen. Speziell luftperforierte Kautschukund Acrylatklebeschichten zeigen ein solches Verhalten.

Geeignet sind Klebeschichten, die per se wasserdampfdurchlässig sind, Poren enthalten, die sich während der Anwendung nicht schließen können, oder in die feuchtigkeitsabsorbierende Füllstoffe eingearbeitet sind.

Denkbar wären auch Hydrogelklebeschichten. Problematisch ist jedoch, dass sie befeuchtend wirken und somit durch ihre Anwendung auf der Haut zur Mazeration führen können.

Als Klebeschichten werden daher insbesondere Polyurethanklebeschichten, Silikonklebeschichten oder partiell vernetzte Hydrokolloidklebeschichten bevorzugt, die zusammen mit dem Träger eine Wasserdampfdurchlässigkeit von mind. 300 g/m² in 24 h, bevorzugt 500 g/m² in 24 h aufweisen und/oder mindestens eben diese Mengen an Feuchtigkeit von der Hautoberfläche innerhalb dieses Zeitraumes aufnehmen und in der Klebeschicht und oder dem Träger zu binden vermögen.

Weiterhin sollte die Klebeschicht bei beiden Varianten bevorzugt trotz hoher Klebkraft so beschaffen sein, dass an den Rändern des Verbandes anhaftender Schmutz ohne viel Aufwand zu entfernen und die Oberfläche zu desinfizieren ist. Dies ist besonders wichtig, da durch die erheblichen Stärken der Klebeschichten (150 - 3000 µm) speziell beim Stanzen von Filmen aus flächigem Material die einzelnen Filmabschnitte einen relativ hohen klebenden Rand aufweisen, an dem Schmutz haften bleiben kann. Das leichte Ablösen anhaftenden Schmutzes ist faktisch möglich, wenn die Klebeschicht, wie erfindungsgemäss bevorzugt, nur geringfügigen oder keinen kalten Fluss zeigt, so dass anhaftender Schmutz nur oberflächlich haftet und nicht von der Klebeschicht umflossen werden kann und bei feuchter mechanischer Reinigung, wie sie beispielsweise bei der Desinfektion der Zitzen üblich, leicht abzulösen ist.

Im Falle einer Verletzung der Haut wird durch das Auflegen und Abnehmen des Films zusätzlich zum Melkvorgang jedes Mal die Wundruhe gestört und ein ggf. bestehender Schorf in aller Regel wieder mit der Entfernung des Verbandes abgerissen. Hierdurch wird die Wundheilung ganz erheblich verzögert und verhindert eine schnelle Wiedereingliederung in den standardisierten Ablauf, was den wirtschaftlichen Schaden weiter erhöht.

Daher ist weiterhin bevorzugt vorgesehen, dass die erfindungsgemäße Klebeschicht auf der Umgebungshaut einer Wunde gut haftet, in Kontakt mit feuchter Wundoberfläche aber nicht klebt. Vielmehr erhält sie stattdessen ein Feuchtigkeitsmilieu auf der Wundoberfläche vergleichbar dem unter einem natürlichen Schorf. Der Film verklebt so nicht im Wundbett und fördert damit eine beschleunigte Abheilung der Wunde. Idealerweise ist dies ein rein physikalischer Effekt, der ohne den Zusatz von Wirkstoffen beobachtet werden kann. Es schließt aber keinesfalls den Einsatz von Wirkstoffen mit z.B. desinfizierender (z.B. Silberionen), heilender (z.B. Dexpanthenol) oder schmerzstillender (z.B. Lidocain) Wirkung aus, die üblicherweise bei derartigen Verletzungen angewendet werden und die in den Film, insbesondere die Klebeschichteingebracht oder auf ihn aufgebracht worden sind.

Die Klebeschicht sollte darüber hinaus ein möglichst geringes Sensibilisierungspotential aufweisen.

Auch die Sauerstoffdurchlässigkeit des Films ist eine vorteilhafte Größe, damit auch die mehrere Tage abgedeckte Haut auch durch den Verband hindurch weiterhin atmen kann. Andernfalls könnte die Zitzenhaut hierdurch gereizt werden und das Tier versuchen, den Film abzulecken. Dies trägt mit dazu bei, dass man den Film längere Tage auf der Zitze belassen kann, was wiederum den Schutz der Zitze erhöht.

Wie oben bereits ausgeführt, ist das erfindungsgemäße Verfahren bzw der Film dazu gedacht, auch verletzte Hautbereiche einer Zitze während des Melkvorganges zu schützen. Um hier den Heilungsverlauf beobachten zu können, sieht eine weitere vorteilhafte Ausgestaltung der Erfindung vor, dass Film und Klebeschicht transparent ausgebildet sind, sodass der Anwender in der Lage ist, den Zustand der Zitze durch den Film hindurch inspizieren zu können.

Eine weitere erfindungsgemäße Ausführung sieht vor, dass angezeigt wird, wann die Wunde unter dem Verband weitgehend abgeheilt ist. Werden beispielsweise in die Klebeschicht Partikel eingearbeitet, die in der Lage sind Flüssigkeit zu binden, so verändern diese Partikel durch die Aufnahme von Flüssigkeit ihre Korngröße und ihren Brechungsindex, wobei der an sich transparente Film an der Stelle, an der der Film die Flüssigkeit aufgenommen hat, milchig trüb wird. Ist nun der Film in seiner Gesamtheit wasserdampfdurchlässig, so wird die in den Partikeln zwischenzeitlich gebundene Feuchtigkeit über die von der Haut abgewandten Seite des Film an die Umgebung abgegeben. Die Feuchtigkeit wird, wie zuvor bereits beschrieben, durch den Film hindurch transportiert. Solange Wundsekret und damit Feuchtigkeit aus der Wunde nachgeliefert wird, bleibt der Verband an der Stelle, unter der sich der die Wunde befindet, milchig trüb. Sobald sich allerdings neues Epithel gebildet hat wird keine Körperflüssigkeit vom Körper mehr nachgeliefert. Dennoch wird bereits im Verband gebundene Feuchtigkeit weiterhin an die Umgebung abgegeben, wobei der Verband langsam seine milchige Eintrübung verliert und wieder transparent zu werden beginnt. Dies kann als sicheres Zeichen dafür gewertet werden, dass die durch den Verband abgedeckte Wunde abgeheilt ist und der Verband entfernt werden kann.

Ein erfindungsgemäß ausgestalteter Verband verändert sich optisch, wenn er mit Wundsekret in Berührung kommt und dieses aufnimmt, sodass er sich deutlich von Bereichen, die nur mit der Haut in Berührung kommen, unterscheidet. Diese optische Veränderung bildet sich zurück, sobald kein Wundsekret mehr fließt.

In Verbindung hiermit ist von wesentlicher Bedeutung, dass ein erfindungsgemäßer Film aufgenommenes Wundsekret unter Druck nicht wieder abgibt, sprich, dass er durch den Melkvorgang ausgeübte Druck nicht ausreicht, um Wundsekret aus dem Film herauszudrücken. Dies kann man erreichen, indem man der Klebstoffschicht Superabsorber-Partikel beimischt, wie sie z.B. aus der Windelherstellung bekannt sind. Geeignete Partikel sind z.B. unter dem Namen FAVOR T 5233 im Handel erhältlich.

Weiterhin ist schließlich vorgesehen, dass der Film farblich gestaltet oder gekennzeichnet sein kann. Auf diese Weise kann der Tierhalter bei Bedarf die Tiere im Stall schnell identifizieren, die versorgt worden sind und damit unter besonderer Beobachtung stehen, auch wenn sie weiterhin mit dem Rest der Herde gehalten werden.

Optimiert werden können das erfindungsgemässe Verfahren bzw. der erfindungsgemässe Film durch vorteilhafte Ausgestaltungen des Trägers.

Grundsätzlich geeignete Träger können alle aus allen flexiblen flächigen Materialien bestehen, die einen flächigen Zuschnitt erlauben und z.B. die aus dem Gebiet der Pflaster- oder Verbandtechnik bekannten Eigenschaften aufweisen. Hier zum Einsatz kommende Träger sind beispielsweise Gewebe, Gewirke, Vliese und Folien sowie Kombinationen aus den Materialien. Geeignete Folien sind z.B. Polyethylenfolien, Polyurethanfolien, Copolyesterfolien, Polyamidfolien und Coextrudierte Folien. Geeignete Vliese sind z.B. Celluloseacetate, Polyestervliese oder Polyamidvliese. Es handelt sich bei den vorstehenden Angaben um eine nicht abschließende Aufzählung einiger Beispiele. Selbstverständlich sind noch eine Reihe weiterer dem Fachmann bekannte Materialien zur Herstellung als Träger geeignet.

Gemäss einer vorteilhaften Ausgestaltung wird ein Träger eingesetzt, der seinerseits ebenfalls in Richtung der Zitze dehnbar ausgebildet ist. Die Dehnung sollte so gewählt sein, dass der Träger mit der Klebeschicht sich bei üblichen in Melkgeschirren auftretenden Kräften in Richtung der Längsachse der Zitze auf mindestens auf > 50 % bevorzugt > 100% seiner Erstreckung in dieser Richtung dehnen lässt. Besonders bevorzugt ist, wenn sich Träger und Klebeschicht im wesentlichen synchron entsprechend der Längung der Zitze dehnen lassen.

Die Dehnung des Trägers kann sowohl durch Recken als auch elastisches Dehnen mit allen dazwischen liegenden prozentualen Verhältnissen zwischen Reckung und Elastizität erfolgen.

Bevorzugt ist vorgesehen, dass der Träger auch quer zur Längsachse der Zitze dehnbar ist. Auch hier kann die Dehnung wieder auf Elastizität oder Reckung zurückzuführen sein.

Als Faustregel kann man festhalten, dass eine Kraft von ca. 50 kPa in der Lage ist, die Zitze einer Kuh auf einen zum Melken erforderlichen Wert von 140-150% der ursprünglichen Länge zu dehnen. In etwa bei dieser Kraft sollte auch die Dehnung des Trägers auf ähnliche Längen erfolgen. Dies sind nur Beispielswerte. Je nach Melksystem und gemolkenem Tier können die Werte erheblich abweichen, wobei die jeweiligen auftretenden Kräfte dem Fachmann hinlänglich bekannt sind.

Die der Klebeschicht abgewandte Oberfläche des Trägers sollte so beschaffen sein, dass im Stall üblicher Schmutz nicht oder nur in geringem Maße an der Oberfläche des Trägers haften oder in ihn eindringen kann. Geringfügig anhaftender Schmutz muss sich problemlos entfernen lassen. Auch muss die Oberfläche mit gängigen Mitteln desinfizierbar sein, sodass nach der Entfernung von grobem Schmutz eventuell noch an der Oberfläche vorhandene Keime abgetötet werden können. Als Träger bevorzugt sind daher makroskopisch glatte Folien wie z.B. Polyurethan-, Polyethylen oder Polyesterfolien oder Verbünde aus mehreren unterschiedlichen Materialien, die sichtbar glatt, keimdicht und flexibel sind. Weniger geeignet, aber gleichfalls möglich sind aus diesem Grunde stärker strukturierte Oberflächen wie z.B. Gewirke, Gewebe oder Vliese, auch wenn sie hydrophobiert sind.

Der Träger ist idealerweise selbst oder in Verbindung mit der Klebeschicht keimdicht und bleibt dies auch bei Dehnung bzw. Reckung.

Vorteilhaft vorgesehen ist, dass der Träger durch die stetige Druck- und Zugbelastung, die sich im Millisekundenbereich ändert und mehr als einmal pro Sekunde einen kompletten Cyclus durchläuft, nicht plastisch verformt wird und stets wieder in der Lage ist seine ursprüngliche Form anzunehmen. Sollte im Extremfall nur die Klebeschicht elastisch, der Träger aber nur reckbar sein, so entstehen durch die Rückstellkraft der Klebeschicht Falten im abdeckenden Träger, die die Funktionsfähigkeit des Films zwar nicht beeinträchtigen, jedoch nicht ideal sind, da sich darin zusätzlich Schmutz verfangen kann.

Erfindungswesentlich ist dass der Film überlappend und auf sich selbst haftend angelegt wird. Wichtig ist in diesem Zusammenhang, dass die Klebeschicht nicht nur auf der Haut haftet sondern auch auf der nach aussen weisenden Oberfläche des Trägers. Die Haftverbindung sollte so fest sein, damit sich der Film nicht von selbst oder durch mechanische Einflüsse wie z.B. das Melken oder Schlecken der Tiere trennt und abwickelt.

In einer vorteilhaften Ausgestaltung ist vorgesehen, dass der Film mindestens 5 % bezogen auf den Zitzenumfang mit sich selbst überlappt. Es ist insbesondere bei dieser Ausgestaltung wichtig, dass die Klebeschicht mindestens so gut wie auf der Haut auf dem sie abdeckenden Träger haftet.

Im Rahmen der Erfindung kann der Film aber auch mehrfach um die Zitze gewickelt werden. Man kann so einerseits einen besonders guten Schutz von gestressten Hautbereichen erreichen. Andererseits lassen sich auf diese Weise Zitzen mit zu geringem Durchmesser problemlos an vorhandene Melkgeschirre anpassen, was überhaupt erst das Melken solcher vom Herdenstandard abweichender zu kleiner Zitzen möglich macht.

Ein besonders bevorzugter Film weist einen Träger auf, beschichtet mit einer selbstklebenden Polymermatrix, die keinen oder nur geringfügigen kalten Fluss zeigt. Als Beispiel einer für die Anwendung relevanten Ausführungsform einer Klebeschicht gelten Qualitäten, wie sie in Patent EP 0 897 406 hinreichend beschrieben sind. Der für die erfindungsgemäße Anwendung bevorzugte Vernetzungsgrad, charakterisiert als Isocyanatkennzahl wie beschrieben in Patent EP 0 897 406, liegt bevorzug in Bereich von 41- 47, ohne jedoch die im vorgenannten Patent genannten Kennzahlbereiche ausschließen zu wollen. Der in diesem Patent beschriebenen Polymermatrix werden bevorzugt 5 - 20 % eines Füllstoffes bezogen auf die eingesetzte Menge an Polyol zugesetzt, der in der Lage ist wässrige Flüssigkeiten zu absorbieren und zu binden, ohne jedoch die im vorgenannten Patent sowie in Patent EP 0 665 856 genannten Füllstoffmengen und -Qualitäten ausschließen zu wollen. Die so charakterisierte Klebeschicht ist mit einem hochflexiblen, wasserdampf und sauerstoffdurchlässigem Polyurethanfilm abgedeckt, ohne jedoch die in den vorgenannten Patenten genannten alternativen Filme ausschließen zu wollen. Abgedeckt wird das Produkt mit handelsüblichem, dem Fachmann für selbstklebende Produkte geläufigen Trennpapieren oder Trennfolien, die vor der Anwendung entfernt werden müssen, um die Klebeschichtenseite bis zum Zeitpunkt der Anwendung zu schützen.

Das Produkt für die erfindungsgemäße Anwendung kann aus flächigem Material heraus gestanzt oder geschnitten als Einzelpflaster oder von einer Rolle abgeschnitten angewendet werden. Letzteres ermöglicht durch Abschneiden eines der Zitzengröße angemessenen Pflasterstücks die einfache Anpassung an unterschiedliche Zitzenumfänge. Pflaster können aber auch einzeln gegossen werden-Beispielhaft seien Verfahren genannt, wie sie in der Patentanmeldung EP 1 695 721 beschrieben sind.

Bevorzugt kann vorgesehen sein, dass der Zuschnitt an den abzudeckenden Hautbereich der Zitze angepasst geformt ist. Generell weisen Zitzen eine koni-sche sich nach unten hin zunehmend verjüngende Form auf.

In einer Ausgestaltung der Erfindung ist daher vorgesehen dass der Zuschnitt in Form eines bogenförmigen Streifens mit an die Konizität der Zitze angepasstem Radius ausgebildet ist. Es können gemäß dieser Ausgestaltung unterschiedliche Zuschnitte für unterschiedliche Höhenbereiche der Zitze vorgesehen werden. Im oberen, eher zylindrischen Bereich der Zitze können für eine optimale Fixierung bevorzugt gering gebogene Zuschnitte eingesetzt werden, während im unteren sich stärker verjüngenden Bereich Zuschnitte stärker gebogene Zuschnitte besser, d.h. ohne z.B. Falten zu werfen, aufgebracht werden können. Denkbar ist z.B. ein Set von Zuschnitten mit unterschiedlich stark gekrümmten bogenförmigen Streifen vorzusehen, aus dem der Anwender je nach abzudeckendem Zitzenbereich den am besten geeigneten auswählen kann.

Weitere Ausgestaltungen betreffen den Spitzenbereich der Zitze. In diesem Zusammenhang ist bevorzugt vorgesehen, dass der den Film bildende Zuschnitt ein durchgehendes Loch umgibt, dessen Durchmesser mindestens dem Durchmesser der Austrittsöffnung eines in der Zitze verlaufenden Milchkanals entspricht. Der Film lässt sich damit optimal auf die Spitze einer Zitze und den daran angrenzenden Zitzenbereich aufkleben, wobei lediglich Sorge getragen werden muss, dass das in dem Zuschnitt vorgesehene Loch fluchtend zu der Austrittsanordnung des Milchkanals ausgerichtet wird. Mit einem solchen Film lässt sich in besonders einfacher Weise der Spitzenbereich einer Zitze unter Erhaltung der Melkfähigkeit abkleben.

Optimieren lässt sich diese Ausgestaltung durch die Gestaltung von Zuschnitten, die besonders gut an den Oberflächenverlauf der Zitzenspitze angepasst sind und sich so ohne überstehende Kanten beziehungsweise Falten aufkleben lassen.

Denkbar ist z.B. ein Zuschnitt, in dem im Bereich des Lochs vom seitlichen Rand auf das Loch zulaufende Einschnitte vorgesehen sind. Die Einschnitte ermöglichen, dass der Zuschnitt sich von der Zitzenspitze nach oben und um den an die Spitze angrenzenden Endbereich der Zitze herum verkleben lassen, ohne dass es zu Falten oder Ausstülpungen kommt. Optimieren lässt sich der Zuschnitt über die Anzahl der Einschnitte bzw. ihre Form. Denkbar ist z.B., dass die Einschnitte sich nach aussen hin V-förmig aufweiten.

In einer weiteren Ausgestaltung weist der Zuschnitt einen das Loch umgebenden zentralen Abschnitt auf, von dem aus sich mindestens ein streifenförmiger Abschnitt radial zum Loch erstreckt. Besonders vorteilhaft sind mehrere sich radial erstreckende streifenförmige Abschnitte vorgesehen. Ein so ausgestaltetes Zitzenpflaster wird mit seinem zentralen Bereich auf die Zitzenspitze derart aufgeklebt, dass das Loch mit der Austrittsöffnung des Milchkanals fluchtet. Dann werden die sich radial erstreckenden Streifen in Richtung ihrer Erstreckung nach oben an die Zitze angelegt und mit dieser verklebt. Bei dieser Ausgestaltung ist die Spitze der Zitze vollständig -ausgenommen der Bereich des Austrittskanalsund der daran angrenzende Endbereich der Zitze teilweise von dem Film abgedeckt, da zwischen den streifenförmigen Abschnitten freie Räume verbleiben. Je nach Art beziehungsweise Ort der Verletzung und Hautreizung kann dies ausreichen.

Ist jedoch eine vollständige Abdeckung gewünscht, so sieht eine weitere Ausgestaltung vor, dass an mindestens einem der sich radial von dem Loch erstreckenden Streifen des Zuschnitts weitere seitlich abgehende Abschnitte vorgesehen sind. Diese Abschnitte können zum Beispiel die Form eines Streifens mit gewünschter Breite haben.

Die Applikation des Films erfolgt wie oben beschrieben. Zunächst wird zum Beispiel wiederum der zentrale das Loch umgebende Abschnitt auf die Zitzenspitze aufgeklebt. Dann werden die oder der sich radial erstreckende Streifen nach oben in Richtung ihrer Erstreckung an die Zitze gelegt und mit dieser verklebt. Anschließend werden die seitlich von diesem Streifen abgehenden Abschnitte um die Zitze gelegt und auch mit dieser verklebt. Auf diese Weise kann ein unmittelbar an die Zitzenspitze angrenzender Endbereich der Zitze vollständig von dem Film abgedeckt werden, wobei keine Knicke oder Falten auftreten.

In einer weiteren Ausgestaltung ist vorgesehen, dass der Film mindestens einen zur temporären Abdeckung des Loches zwischen den Melkvorgängen geeigneten Klebestreifen aufweist. Idealerweise handelt es sich dabei um einen beziehungsweise mehrere separate Klebestreifen, die jeweils neu nach dem Melkvorgang zum Schließen des Loches auf den Film aufgeklebt werden.

Überraschend für den Fachmann ist, dass im Rahmen der Erfindung beschriebenen Filme bei der hohen Belastung, der das Material beim Reinigungs- und Melkprozess der Zitze sowie den hygienischen Bedingungen einer Milchviehhaltung ausgesetzt ist, sicher sitzen, nicht verrutschen und mehrere Tage auf der Zitze verbleiben, ohne Tendenz zu zeigen, sich abzulösen. Dies ist sowohl gegeben beim Handmelken, beim Einsatz halbautomatischer Melkmaschinen sowie beim Einsatz vollautomatischer Melkroboter. Selbst beim Extrembeispiel Melkroboter wird sowohl die Desinfektion, das Reinigen mit rotierenden Bürsten, das Melken als auch die Nachreinigung problemlos mehrfach überstanden. Zusammengefasst wird dies im Wesentlichen erreicht durch den Einsatz eines flexiblen Trägers, der mit einer flexiblen Klebeschicht beschichtet ist, die keinen kalten Fluss aufweist, wobei der Film überlappend aufgeklebt ist , so dass er zu einem gewissen Teil auf sich selbst klebt.

Überraschenderweise lässt sich ein solcher Film auch trotz der exzellenten Haftung auf der Zitze durch Abrollen leicht entfernen. Weiterhin überraschend zeigen die Tiere, die den Film tragen, nur geringe Tendenz den Film abzuschlecken.

Angewendet werden kann das erfindungsgemässe Verfahren bzw. der Film bei allen Nutztieren, die zur Milchproduktkion herangezogen werden, wie z.B. Kühe, Schafe, Ziegen, Yaks, Kamele, Pferde etc.

Die Erfindung ist nicht nur auf das angesprochene Verfahren bzw. die in dem Verfahren einsetzbaren Filme gerichtet. Sie bezieht sich auch auf die Verwendung solcher Filme zum Schutz von Hautbereichen im Zitzenendbereich während des Melkvorganges.

Im Folgenden soll die Erfindung anhand eines Beispiels für einen geeigneten Film und mehrerer Figuren näher erläutert werden.
- Fig. 1: zeigt schematisch eine in einem Melkbecher angeordnete Zitze mit aufgeklebtem Film
- Fig. 2: zeigt einen Zuschnitt für einen für den oberen Bereich der Zitze gedachten Film in Form eines bogenförmigen Streifens
- Fig. 3: zeigt einen Schnitt durch das für die Fertigung des erfindungsgemäßen Films eingesetzte Verbundmaterial.
- Figs.4 bis 8: zeigen unterschiedliche Ausführungsformen von Zuschnitten von für die Zitzenspitze gedachten Filmen.
- Fig. 9: zeigt den Endbereich einer Zitze mit aufgeklebtem Film.
- Fig. 10: zeigt die Ansicht aus Fig. 9 in einer Längsschnittansicht.

### BEISPIEL:

Ein geeigneter Film ist aufgebaut aus einer etwa 40 µm starken hochflexiblen, wasserdampf- und sauerstoffdurchlässigen sowie keimdichten Polyurethanfolie (Applica, Smith & Nephew) als Träger, der beschichtet ist mit einer selbstklebenden beispielsweise 300 µm oder 800 µm dicken hochflexiblen Polyurethanklebeschicht.

Die Klebeschicht wurde hergestellt, indem man 100 Gew.-Anteile an Polyol (Levagel VP KA 8732; OH-Zahl 35) mit 12 Gew.-Teilen an Superabsorber (Favor T 5233), 0,1 Gew.-Teilen Katalysator (Coscat 83) und 0,8 Gew.-Teilen Vitamin E (Irganox E 201) in einer 1-1-Apparatur 2 h bei Raumtemperatur homogenisiert. Zu 100 Gew.-Teilen dieser Mischung wurden dann 6,6 Gew.-Teile an Vernetzer (Desmodur E 305, NCO-Gehalt 12,2 %) gegeben und diese intensiv mit einem Glasstab 1 Minute vermischt.

Diese Mischung wird dann auf ein handelsübliches, silikonisiertes Trennpapier gegossen, mit der als Träger dienenden Polyurethanfolie abgedeckt und mit Hilfe eines Rakels flächig verteilt, sodass der Verbund eine Dicke von etwa 300 µm bzw. 800 µm aufweist und dann in einem Trockenschrank 18 Minuten bei 80°C gehärtet. Es entsteht ein transparenter Film.

Aus dem entstehenden flächigen Gebilde werden Streifen von z.B. 4 cm Breite herausgeschnitten. Die Breite kann natürlich variieren und abhängig von der Länge der Zitzen gewählt werden. Ein Streifen mit einer Breite von 4 cm ist beispielweise geeignet für eine Zitzenlänge von 4,5 -6 cm.

Ist die Zitze nun deutlich länger als die 4,5 - 6 cm, so kann entweder ein Streiefen mit größerer Breite zugeschnitten werden oder man umwickelt mit einem 4 cm breiten Streifen erst den unterer Teil der Zitze und anschließend den oberen Teil oder umgekehrt, wobei die beiden Wicklungen sich überlappen müssen.

Die Länge des Streifens sollte bei einem üblichen Zitzendurchmesser von 2,6 cm mit etwa etwa 12 cm gewählt werden. Das Trennpapier wird abgezogen und der Film ohne Spannung um die Zitze herum gelegt und angedrückt, sodass der Film die Zitze einmal vollständig umfängt und zu etwa 50 % noch auf sich selbst klebt.

Der Film kann nun einmal so angelegt über mehrere Tage auf der Zitze verbleiben, vor dem Melken gereinigt werden, das Melkgeschirr angelegt, das Tier gemolken und das Melkgeschirr wieder abgenommen werden, ohne das der Verband sich löst.

Ist die Zitze zu dünn, um mit dem Standardmelkzeug für die Herde gemolken zu werden, kann ein entsprechend längeres Stück mehrfach um die Zitze herumgewickelt werden. So kann mit drei Schichten des Films der Zitzendurchmesser z.B. von 2,6 auf 2,8 cm vergrößert werden. Das Melkzeug haftet im vorliegenden Beispiel dann wieder sicher.

Da der Film transparent ausgebildet ist, kann der Anwender jederzeit die Zitzenhaut durch den Verband hindurch inspizieren.

Wenn die Zitze eine Verletzung aufweist, so wird der Film, genau wie oben beschrieben, angelegt. Hierbei übernimmt der Film die Funktion eines Schorfes und schützt die Wunde vor Verunreinigung und Keimen. Überschüssige Wundflüssigkeit wird langsam durch den Film absorbiert. An der Stelle, an der der Film das Sekret absorbiert, verliert der Film seine Transparenz und wird milchig trüb. Auch jetzt kann noch gemolken werden. Wundsekret wird nicht aus dem Film herausgepresst und das Euterviertel muss nicht trockengestellt werden. Ist die Wunde unter dem Film abgeheilt, so verliert der Film sein milchig-trübes Aussehen wieder und wird transparent. Der Film kann nun, wenn er zur Versorgung der Wunde aufgebracht worden ist, einfach und praktisch ohne Kraftaufwand wieder entfernt werden. Dies erfolgt durch einfaches Abrollen von der Zitze von oben nach unten.

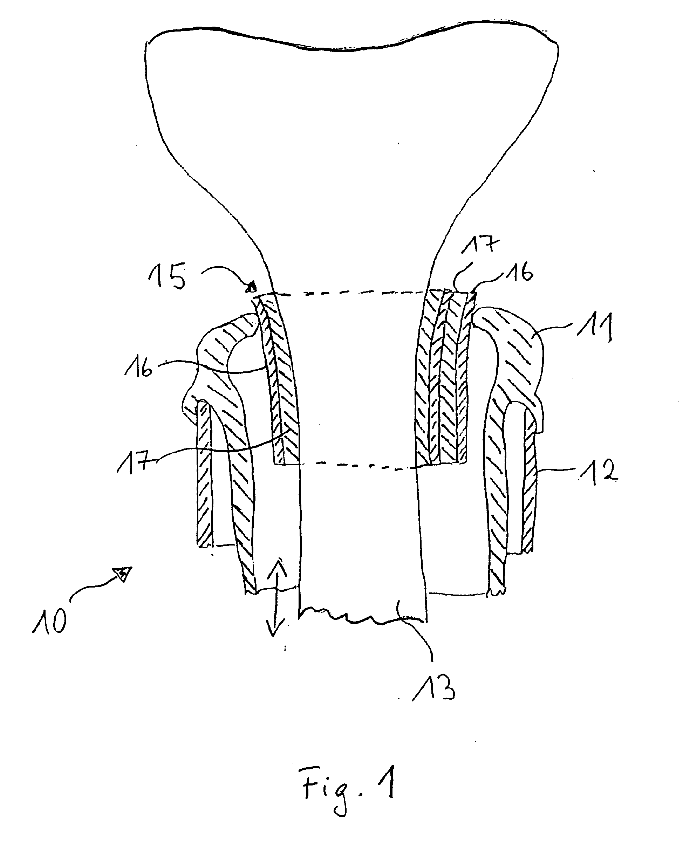

In Fig. 1 dargestellt ist schematisch ein Euter mit einer Zitze 13. Die Zitze ist in einem üblichen Melkbecher 10 aufgenommen, der aus einer äußeren Stahlhülse 12 und einem darauf aufgesetzten Zitzengummi 11 besteht.

Durch abwechselndes Anlegen von Vakuum in dem Zitzengummi bzw. dem den Zitzengummi umgebenden Bereich zwischen Gummi 11 und Hülse 12 wird einerseits der Melkvorgang und andererseits eine Massage der Zitze durchgeführt. Diese Vorgänge sind dem Fachmann bekannt. Es wird an dieser Stelle nicht weiter darauf eingegangen.

Wie schematisch angedeutet, ist die Zitze 13 links einlagig und rechts zweilagig mit einem Film 15 umwickelt. Der Film 15 ist ein Verbund aus einem Träger 16 und einer Klebeschicht 17, mit der er auf sich selbst haftet auf der Zitze 13 befestigt ist.

Während des Melkvorganges wird die Zitze 13 in Richtung des Pfeils nach unten gestreckt.

Erfindungsgemäß ist vorgesehen, dass die Klebeschicht 17 entweder diese Dehnung toleriert, also mitmacht, oder aber in dem Moment, wo über die nach unten gedehnte Zitze eine Kraft auf die Klebeschicht 17 ausgeübt wird, tangential abschert, wobei sie beim nächsten Andruck durch den Zitzengummi wieder ausreichend fest verklebt.

Im ersten Fall ist vorgesehen, dass der Träger 16 die Dehnung vorteilhafterweise ebenfalls mit ausführt, da ansonsten Scherkräfte über die Klebeschicht auf die Zitze wirken würden. Im zweiten Fall ist dies nicht erforderlich.

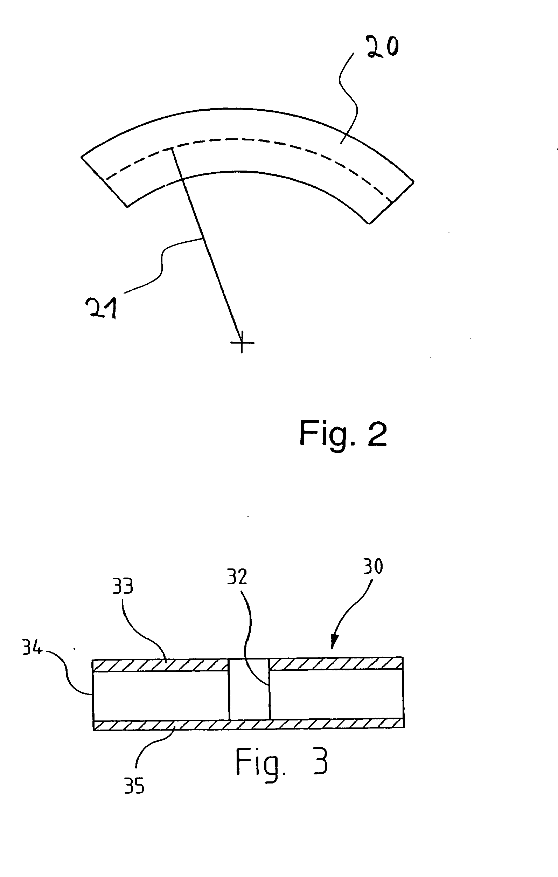

Fig. 2 zeigt einen Ausführung eines Zuschnitts 20 für einen Film, der besonders für den Schaftbereich der Zitze geeignet ist. Der Zuschnitt 20 hat die Form eines bogenförmigen Streifens mit einem Radius 21, der so gewählt ist, dass der Streifen optimal auf der konusförmigen Zitze aufgeklebt werden kann. Es versteht sich, dass Zuschnitte mit unterschiedlichen Radien in einem Set bereitgestellt werden können, aus denen der Anwender in Abhängigkeit von der Zitzenform den am besten geeigneten auswählt.

Fig. 3 zeigt den grundsätzlichen Aufbau eines im Rahmen der Erfindung einsetzbaren Films im Schnitt. Der Film 30 weist von oben nach unten gesehen einen Träger 33, eine Klebeschicht 34 und eine vor Aufbringung des Films zu entfernende Abdeckung 35 auf. Im hier gezeigten Fall weist der Film noch zusätzlich ein Loch 32 auf, das wie oben erwähnt und in den nachfolgenden Ausführungen dargestellt, für eine Anwendung im Bereich der Zitzenspitze erforderlich ist. Es versteht sich, dass bei Filmen die nicht in diesem Bereich der Zitze aufgebracht werden sollen, auf das Loch 32 verzichtet werden kann.

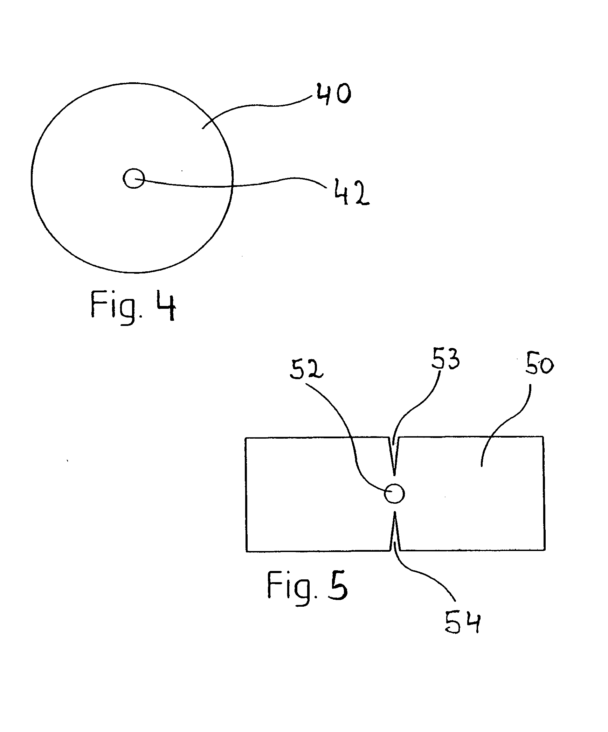

Die Figuren 4-10 zeigen Ausführungen, die spezielle für den Spitzenbereich der Zitze gedachte Filme betreffen.

Fig. 4 zeigt in Aufsicht einen kreisrunden Zuschnitt 40, der ein Loch 42 umgibt. Dies ist die einfachste Ausführungsform, die allerdings nicht unter allen Umständen optimal verklebbar ist.

Fig. 5 zeigt in Aufsicht einen weiteren Zuschnitt 50 des erfindungsgemäßen Zitzenpflasters. Auch dieser Zuschnitt 50 umgibt ein Loch 52. Weiterhin sind vom Rand des Zuschnitts 50 auf das Loch 52 hin verlaufende Einschnitte 53 und 54 in dem Zuschnitt 50 vorgesehen. Diese Einschnitte sollen ein Verkleben des Zuschnitts 50 mit der Zitzenspitze erleichtern. Es versteht sich, dass solche Einschnitte selbstverständlich auch in dem in Fig. 4 gezeigten Zuschnitt oder anderen Zuschnittsformen ausgebildet werden können und dort denselben Effekt haben. Selbstverständlich ist es auch möglich noch mehr Einschnitte vorzusehen, oder die Einschnitte anders zu gestalten, z.B. sich V- oder U-förmig nach aussen aufweitend.

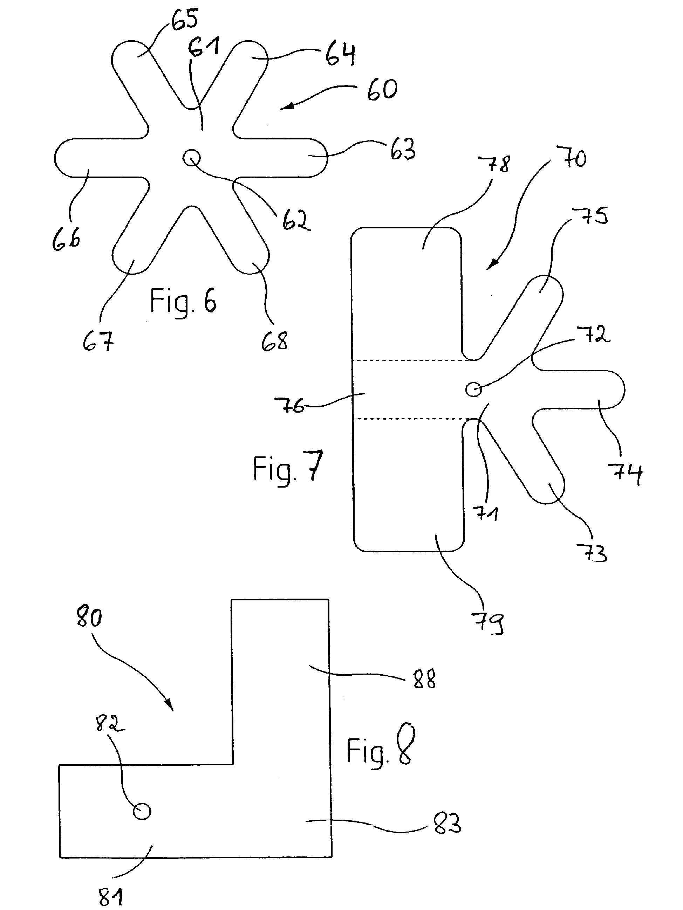

Fig. 6 zeigt eine weitere Ausführung eines Zuschnittes 60 für ein erfindungsgemäßes Zitzenpflaster. Der Zuschnitt 60 weist einen zentralen Abschnitt 61 auf, der ein Loch 62 umgibt. Weiterhin vorgesehen sind sich vom zentralen Abschnitt 61 radial zum Loch 62 erstreckende Streifen 63 bis 68. Beim Anbringen des Pflasters werden diese Streifen 63 bis 68 seitlich an der Zitze hochgeführt und dann mit den darunter liegenden Hautbereichen verklebt. Mit einem solchen Zuschnitt ist sichergestellt, dass das verklebte Pflaster keine Falten wirft oder Kanten ausbildet. Andererseits verbleiben im Zitzenendbereich nicht abgedeckte Bereiche.

In diesem Zusammenhang zeigt Fig. 7 eine weitere Ausführungsform eines Zuschnittes 70 für ein erfindungsgemäßes Zitzenpflaster. Auch dieser Zuschnitt weist wiederum einen zentralen Abschnitt 71 auf, der ein Loch 72 umgibt. Wie in der in Fig. 6 gezeigten Ausgestaltung sind auch hier sich radial von dem Loch erstreckende streifenförmige Abschnitte 73, 74, 75 und 76 vorgesehen. An dem streifenförmigen Abschnitt 76 sind weitere, sich zu beiden Seiten dieses Abschnittes erstreckende Abschnitte 78 und 79 vorgesehen. Diese Abschnitte 78 und 79 können seitlich um die Zitze gelegt und mit dieser verklebt werden und decken dann die Zitze in einem definierten Höhenbereich vollständig ab.

Eine weitere Ausführungsform eines Zuschnittes 80 zeigt Fig. 8. Bei dieser Ausführungsform sind ein zentraler Abschnitt 81 sowie sich davon erstreckende streifenförmige Abschnitte 82 und 83 insgesamt in Form eines Streifens ausgebildet. Auch hier ist ein seitlich ansetzender Abschnitt 88 vorgesehen, der wie bei der zuvor besprochenen Ausführungsform eine Umwicklung der Zitze in einem definierten Höhenbereich erlaubt. In dem zentralen Abschnitt 81 ist wie bei allen vorher diskutierten Ausführungsformen ein Loch 82 vorgesehen.

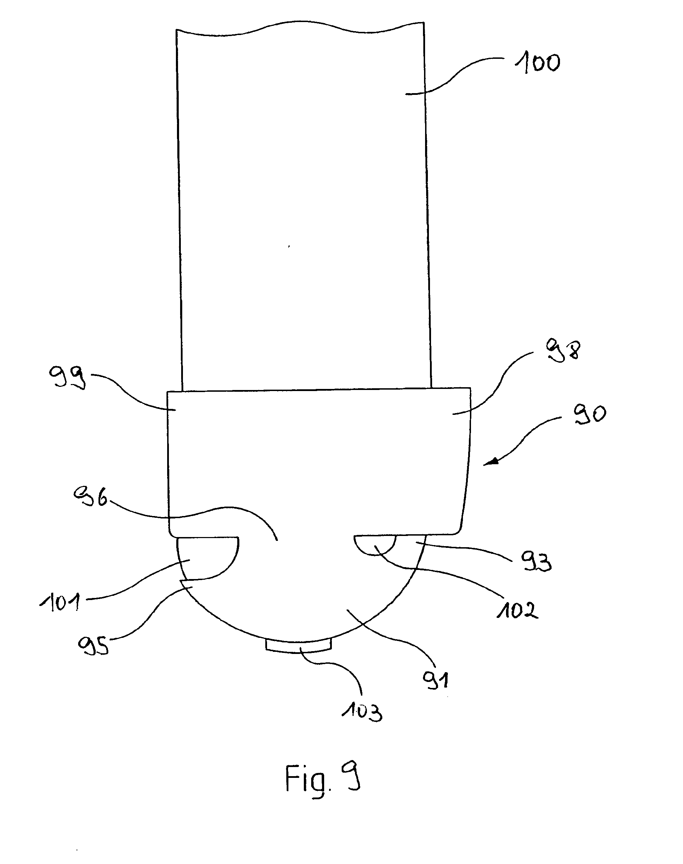

Fig. 9 zeigt einen Endbereich einer Zitze 100 auf den ein Zitzenpflaster mit einem Zuschnitt 90 aufgeklebt ist, der in etwa dem in Fig. 8 gezeigten Zuschnitt entspricht. Der Zuschnitt 90 weist einen zentralen Abschnitt 91 auf, der über die Spitze des Endbereiches der Zitze 100 geklebt ist. Von dem zentralen Bereich 91 erstrecken sich in dieser Darstellung zu erkennenden streifenförmige Abschnitte 93, 95 und 96. An dem streifenförmigen Abschnitt 96 sind seitliche Abschnitte 98 und 99 vorgesehen, die um die Zitze 100 herumgewickelt werden können. Man erkennt, dass die Zitze 100 bis auf Bereiche 101 und 102 nahezu vollständig und faltenfrei durch den Zuschnitt 90 abgeklebt ist. Auch diese Bereiche lassen sich bei entsprechender Optimierung des Zuschnittes noch abdecken.

Schließlich ist in Fig. 9 ein Streifen 103 dargestellt, mit dem das in dieser Darstellung nicht zu erkennende Loch in dem zentralen Abschnitt 91 des Zuschnittes 90 abgedeckt werden kann.

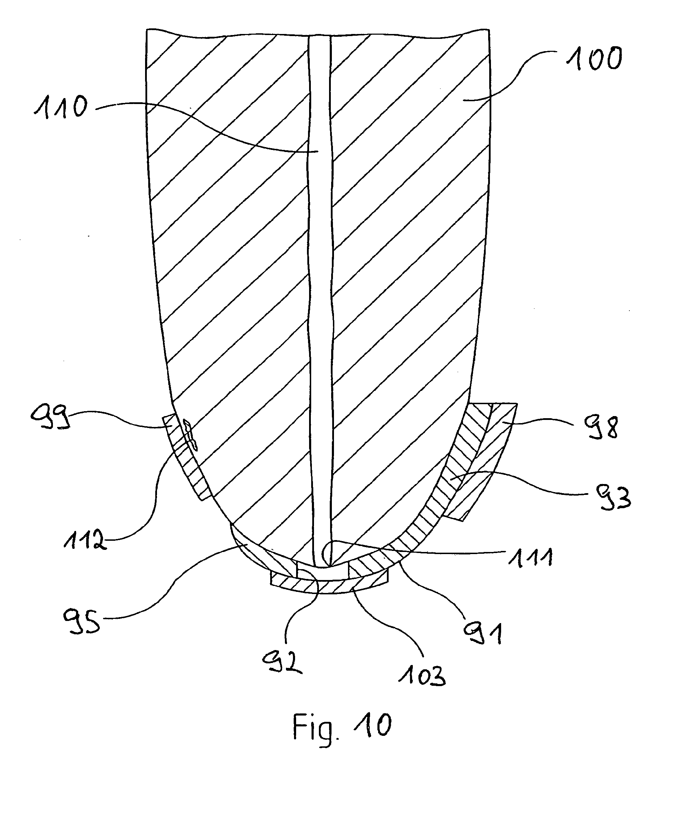

Fig. 10 zeigt die Darstellung aus Fig. 9 in einem Längsschnitt. In dieser Darstellung ist ein durch den Endbereich der Zitze 100 verlaufender Milchkanal 110 zu erkennen, der in einer Austrittsöffnung 111 mündet. Weiterhin ist in dieser Darstellung zu erkennen, dass in dem zentralen Abschnitt 91 des Zuschnittes 90 ein Loch 92 ausgebildet ist, das zu der Austrittsöffnung 111 des Milchkanals 110 fluchtend angeordnet ist.

Von den sich vom zentralen Abschnitt 91 erstreckenden streifenförmigen Abschnitten sind nur die Abschnitte 93 und 95 zu erkennen. Weiterhin sind die seitlichen Abschnitte 98 und 99 dargestellt. Zusätzlich schematisch dargestellt ist eine Wunde 112 im Endbereich der Zitze 100, die von dem seitlichen Abschnitt 99 abgedeckt wird. Weiterhin erkennt man nun, dass der Streifen 103 das Loch 92 abdeckt.

## Patentansprüche

1. Verfahren zum Schutz von Hautbereichen einer Zitze (13, 100) während des Melkvorganges oder zur Anpassung des Zitzendurchmessers an ein Melkgeschirr (10, 11, 12), bei dem die Zitze (13, 100) überlappend mit einem Film umwickelt wird, der ein Zuschnitt (15, 20, 30, 40, 50, 60, 70, 80, 90) aus einem Verbundmaterial aus einem flächigen Träger (16, 33) mit einer Klebschicht (17, 34) ist, über die der Film (15, 20, 30, 40, 50, 60, 70, 80, 90) an sich selbst und an der Haut haftet, wobei die Klebeschicht (17, 34) aus der Gruppe Polyurethanklebeschicht, Silikonklebeschicht, Acrylatklebesrhicht, synthetische Kautschukklebeschicht oder partiell vernetzte Hydrokolloidklebschichten gewählt ist, eine Wasserdampfdurchlässigkeit von mind. 300 g/m² in 24 h, bevorzugt 500 g/m² in 24 h aufweist und mit einer Dicke zwischen 150-3000 µm und einer elastischen und/oder plastischen Verformbarkeit gewählt ist, die bezogen auf einen auf Stahl flächig verklebten Film (15, 20, 30, 40, 50, 60, 70, 80, 90) ein Zurückschieben des Trägers (16, 33) vom Rand zu seiner Mitte um mehr als 500% der Dicke des Klebschicht (17, 34) ermöglicht, derart, dass sie bei Dehnung der Zitze (13, 100) während des Melkvorgangs von dem Film (15, 20, 30, 40, 50, 60, 70, 80, 90) auf die Haut wirkende Scherkräfte kompensiert und/oder die Klebeschicht (17, 34) so gewählt ist, dass sie sich bei einer in Längsrichtung der Zitze (13, 100) auf sie wirkenden Kraft von der Haut ohne Verletzung der Corneozytenschicht ablöst.

2. Verfahren nach Anspruch 1, **dadurch gekennzeichnet, dass** die Klebeschicht (17, 34) dergestalt elastisch ausgebildet ist, dass sie sich nach Wegfall der auf sie wirkenden Scherkräfte wieder im wesentlichen in ihre ursprüngliche Form rückverformt.

3. Verfahren nach einem der vorhergehenden Ansprüche, **dadurch gekennzeichnet, dass** die Zitze (13) mehrfach mit dem Film (15) umwickelt wird.

4. Verfahren nach Anspruch 3, **dadurch gekennzeichnet, dass** der Durchmesser der Zitze durch Umwickeln mit dem Film an ein Melkgeschirr angepasst wird.

5. Film für ein Verfahren gemäss Anspruch 1, **dadurch gekennzeichnet, dass** er auf sich selbst haftend ausgebildet ist, ein Zuschnitt (15, 20, 30, 40, 50, 60, 70, 80, 90) aus einem Verbundmaterial aus einer Klebeschicht (17, 34) mit einem einseitig darauf angeordneten flächigen Träger (16, 33) ist, wobei die Klebeschicht (17, 34) aus der Gruppe Polyurethanklebeschicht, Silikonklebeschicht, Acrylatklebeschicht, synthetische Kautschukklebeschicht oder partiell vernetzte Hydrokolloidklebschichten gewählt ist, eine Wasserdampfdurchlässigkeit von mind. 300 g/m² in 24 h, bevorzugt 500 g/m² in 24 h aufweist und eine Dicke zwischen 150-3000 µm und eine elastische und/oder plastische Verformbarkeit aufweist, die bezogen auf einen auf Stahl flächig verklebten Film (15, 20, 30, 40, 50, 60, 70, 80, 90) ein Zurückschieben des Trägers (16, 33) vom Rand zu seiner Mitte um mehr als 500% der Dicke des Klebschicht ermöglicht und gewährleistet, dass die Klebeschicht (17, 34) bei Dehnung der Zitze (13, 100) während des Melkvorgangs von dem Film (15, 20, 30, 40, 50, 60, 70, 80, 90)auf die Haut wirkende Scherkräfte kompensiert und/oder die Klebeschicht (17, 34) so ausgebildet ist, dass sie die sich bei einer in Längsrichtung der Zitze auf sie wirkenden Kraft von der Haut ohne Verletzung der Corneozytenschicht ablöst.

6. Film nach Anspruch 5, **dadurch gekennzeichnet, dass** er bei üblichen in Melkgeschirren auftretenden Zugkräften um mind. 50% seiner Erstreckung in Richtung der Zitze (13, 100), insbesondere entsprechend der auftretenden Längung der Zitze dehnbar ist.

7. Film nach einem der vorhergehenden Ansprüche 5 oder 6, **dadurch gekennzeichnet, dass** die Klebeschicht (17, 34) und der Träger (16, 33) eine Wasserdampfdurchlässigkeit von mind 300 g/m² in 24h aufweisen, oder die Klebeschicht (17, 34) und/oder der Träger (16, 33) in der Lage ist entsprechende Mengen an Feuchtigkeit zu binden.

8. Film nach einem der vorhergehenden Ansprüche 5 bis 7, **dadurch gekennzeichnet, dass** der Träger und die Klebeschicht transparent sind.

9. Film nach einem der vorhergehenden Ansprüche 5 bis 8, **dadurch gekennzeichnet, dass** der Zuschnitt (15, 20, 30, 40, 50, 80, 70, 80, 90) an den abzudeckenden Hautbereich der Zitze angepasst geformt ist.

10. Film nach Anspruch 9, **dadurch gekennzeichnet, dass** der Zuschnitt (20) in Form eines bogenförmigen Streifens mit an die Konizität der Zitze angepasstem Radius (21) ausgebildet ist.

11. Film nach Anspruch 9, **dadurch gekennzeichnet, dass** der **abzudeckende** Hautbereich die Zitzenspitze ist, und der Zuschnitt (40, 50, 60, 70, 80, 90) ein das Verbundmaterial durchsetzendes Loch (42, 52, 62, 72, 82, 92) umgibt, dessen Durchmesser mindestens dem Durchmesser der Austrittsöffnung (111) eines Milchkanals (110) entspricht.

12. Film nach einem der vorhergehenden Ansprüche, **dadurch gekennzeichnet, dass** eine Abdeckung (103) vorgesehen ist, mit dem sich das Loch (92) von aussen zwischen den Melkvorgängen abdecken lässt.

13. Verwendung eines Filmes gemäss einem der vorhergehenden Ansprüche 5-12 zur Anpassung des Durchmessers einer Zitze an ein Melkgeschirr.

## Claims

1. A method for the protection of skin areas of a teat (13, 100) during the milking process or for adjusting the teat diameter to a milking gear (10, 11, 12) wherein a film that is a cut (15, 20, 30, 40, 50, 60, 70, 80, 90) which is made of a composite material and is cut out from a flat substrate (16, 33) having an adhesive layer (17, 34) is wrapped around the teat (13, 100) in an overlapping manner, with the film (15, 20, 30, 40, 50, 60, 70, 80, 90) adhering to itself and to the skin via said adhesive layer (17, 34), wherein the adhesive layer (17, 34) is selected from the group consisting of polyurethane adhesive layer, silicone adhesive layer, acrylate adhesive layer, synthetic rubber adhesive layer or partially cross-linked hydrocolloid adhesive layers, has a water vapour permeability of at least 300 g/m² in 24 h, preferably 500 g/m² in 24 h, and is selected with a thickness ranging from 150 to 3000 µm and an elastic and/or plastic deformability which, in relation to a film (15, 20, 30, 40, 50, 60, 70, 80, 90) flatly bonded on steel, allows the substrate (16, 33) to be pushed back from the edge to its centre by more than 500 per cent of the thickness of the adhesive layer (17, 34) in such a way that, when the teat (13, 100) is stretched during the milking process, it compensates the shear forces which the film (15, 20, 30, 40, 50, 60, 70, 80, 90) exerts on the skin and/or the adhesive layer (17, 34) is selected such that, when a force is exerted on it in the longitudinal direction of the teat (13, 100), it comes off the skin without injuring the corneocyte layer.

2. The method according to Claim 1, **characterised in that** the adhesive layer (17, 34) is formed elastically in such a way that, after the shear forces exerted on it have ceased to exist, it essentially deforms back to its original shape.

3. The method according to any one of the preceding claims, **characterised in that** the film (15) is wrapped around the teat (13) several times.

4. The method according to Claim 3, **characterised in that** the diameter of the teat is adjusted to a milking gear by the film being wrapped around it.

5. A film for a method according to Claim 1, **characterised in that** it is formed such that it adheres to itself, is a cut (15, 20, 30, 40, 50, 60, 70, 80, 90) which is made of a composite material and is cut out from an adhesive layer (17, 34) having a flat substrate (16, 33) arranged thereon on one side, wherein the adhesive layer (17, 34) is selected from the group consisting of polyurethane adhesive layer, silicone adhesive layer, acrylate adhesive layer, synthetic rubber adhesive layer or partially cross-linked hydrocolloid adhesive layers, has a water vapour permeability of at least 300 g/m² in 24 h, preferably 500 g/m² in 24 h, and has a thickness ranging from 150 to 3000 µm and an elastic and/or plastic deformability which, in relation to a film (15, 20, 30, 40, 50, 60, 70, 80, 90) flatly bonded on steel, allows the substrate (16, 33) to be pushed back from the edge to its centre by more than 500 per cent of the thickness of the adhesive layer and ensures that, when the teat (13, 100) is stretched during the milking process, the adhesive layer (17, 34) compensates the shear forces which the film (15, 20, 30, 40, 50, 60, 70, 80, 90) exerts on the skin and/or the adhesive layer (17, 34) is formed such that, when a force is exerted on it in the longitudinal direction of the teat, it comes off the skin without injuring the corneocyte layer.

6. The film according to Claim 5, **characterised in that**, under the usual tensile forces occurring in milking gears, it can be stretched by at least 50 per cent of its extension in the direction of the teat (13, 100), more particularly according to the elongation of the teat that occurs.

7. The film according to any one of the preceding Claims 5 or 6, **characterised in that** the adhesive layer (17, 34) and the substrate (16, 33) have a water vapour permeability of at least 300 g/m² in 24 h, or the adhesive layer (17, 34) and/or the substrate (16, 33) is/are able to bind appropriate amounts of moisture.

8. The film according to any one of the preceding Claims 5 to 7, **characterised in that** the substrate and the adhesive layer are transparent.

9. The film according to any one of the preceding Claims 5 to 8, **characterised in that** the cut (15, 20, 30, 40, 50, 60, 70, 80, 90) is formed such that it is adjusted to the skin area of the teat that is to be covered.

10. The film according to Claim 9, **characterised in that** the cut (20) is formed in the shape of a curved strip having a radius (21) that is adjusted to the conicity of the teat.

11. The film according to Claim 9, **characterised in that** the skin area to be covered is the teat end and the cut (40, 50, 60, 70, 80, 90) surrounds a hole (42, 52, 62, 72, 82, 92) penetrating the composite material, with the diameter of said hole (42, 52, 62, 72, 82, 92) at least corresponding to the diameter of the exit opening (111) of a milk duct.

12. The film according to any one of the preceding claims, **characterised in that** a cover (103) which can be used to cover the hole (92) from outside between the milking processes is provided.

13. A use of a film according to any one of the preceding Claims 5 to 12 for adjusting the diameter of a teat to a milking gear.

## Revendications

1. Procédé de protection de zones cutanées d'un trayon (13, 100) pendant la traite ou d'adaptation du diamètre du trayon à un gobelet trayeur (10, 11, 12), dans le cadre duquel le trayon (13, 100) est enveloppé, de manière à ce que les bords se chevauchent, d'un film consistant en une pièce découpée (15, 20, 30, 40, 50, 60, 70, 80, 90) dans un matériau composite composé d'un support plan (16, 33) et d'une couche adhésive (17, 34) par l'intermédiaire de laquelle le film (15, 20, 30, 40, 50, 60, 70, 80, 90) adhère à lui même et à la peau, la couche adhésive (17, 34) étant choisie dans le groupe couche adhésive en polyuréthane, couche adhésive en silicone, couche adhésive en acrylate, couche adhésive en caoutchouc synthétique ou couches adhésives hydrocolloïdes partiellement réticulés, et présentant une perméabilité à la vapeur d'eau d'au moins 300 g/m² en 24 h, de préférence 500 g/m² en 24 h et ayant une épaisseur comprise entre 150 et 3000 µm et une déformabilité élastique et/ou plastique qui, pour un film (15, 20, 30, 40, 50, 60, 70, 80, 90) collé sur une surface d'acier, permet un retrait du support (16, 33) du bord vers son milieu sur plus de 500 % de l'épaisseur de la couche adhésive (17, 34), de sorte qu'elle compense, lors de l'extension du trayon (13, 100) pendant la traite, les forces de cisaillement exercées par le film (15, 20, 30, 40, 50, 60, 70, 80, 90) sur la peau, et/ou la couche adhésive (17, 34) étant choisie de façon telle que, sous l'action d'une force s'exerçant sur elle dans le sens longitudinal du trayon (13, 100), elle se détache de la peau sans lésions de la couche cornée.

2. Procédé selon la revendication 1, **caractérisée en ce que** la couche adhésive (17, 34) est de conformation élastique telle qu'elle retrouve essentiellement sa forme d'origine après la disparition des forces de cisaillement agissant sur elle.

3. Procédé selon l'une des revendications précédentes, **caractérisé en ce que** le trayon (13) est enveloppé de plusieurs tours du film (15).

4. Procédé selon la revendication 3, **caractérisée en ce que** le diamètre du trayon est adapté au gobelet trayeur en enveloppant le trayon avec le film.

5. Film pour un procédé selon la revendication 1, **caractérisée en ce qu'**il est réalisé de façon à adhérer à lui-même et consiste en une pièce découpée (15, 20, 30, 40, 50, 60, 70, 80, 90) dans un matériau composite composé d'une couche adhésive (17, 34) avec un support plan (16, 33) placé sur un côté de celle-ci, la couche adhésive (17, 34) étant choisie dans le groupe couche adhésive en polyuréthane, couche adhésive en silicone, couche adhésive en acrylate, couche adhésive en caoutchouc synthétique ou couches adhésives hydrocolloïdes partiellement réticulés, et présentant une perméabilité à la vapeur d'eau d'au moins 300 g/m² en 24 h, de préférence 500 g/m² en 24 h et ayant une épaisseur comprise entre 150 et,3000 µm et une déformabilité élastique et/ou plastique qui, pour un film (15, 20, 30, 40, 50, 60, 70, 80, 90) collé sur une surface d'acier, permet un retrait du support (16, 33) du bord vers son milieu sur plus de 500 % de l'épaisseur de la couche adhésive (17, 34) et garantit que la la couche adhésive (17, 34) compense, lors de l'extension du trayon (13, 100) pendant la traite, les forces de cisaillement exercées par le film (15, 20, 30, 40, 50, 60, 70, 80, 90) sur la peau, et/ou la couche adhésive (17, 34) étant choisie de façon telle que, sous l'action d'une force s'exerçant sur elle dans le sens longitudinal du trayon, elle se détache de la peau sans lésions de la couche cornée.

6. Film selon la revendication 5, **caractérisée en ce que**, en présence des forces de traction produites par les gobelets trayeurs courants, il est extensible d'au moins 50 % de son extension dans le sens du trayon (13, 100), et notamment extensible sur une longueur correspondant à l'allongement du trayon.

7. Film selon l'une des revendications précédentes 5 ou 6, **caractérisé en ce que** la couche adhésive (17, 34) et le support (16, 33) présentent une perméabilité à la vapeur d'eau d'au moins 300 g/m² en 24 h ou que la couche adhésive (17, 34) et/ou le support (16, 33) sont en mesure de lier des quantités correspondantes d'humidité.

8. Film selon l'une des revendications précédentes 5 à 7, **caractérisé en ce que** le support et la couche adhésive sont transparents.

9. Film selon l'une des revendications précédentes 5 à 8, **caractérisé en ce que** la pièce découpée (15,20, 30,40, 50,60, 70, 80, 90) a une forme adaptée à la zone cutanée à recouvrir du trayon.

10. Film selon la revendication 9, **caractérisée en ce que** la pièce découpée (20) a la forme d'une bande arquée dont le rayon (21) est adapté à la conicité du trayon.

11. Film selon la revendication 9, **caractérisée en ce que** la zone cutanée à recouvrir est l'extrémité du trayon et que la pièce découpée (40, 50, 60, 70, 80, 90) entoure un trou (42, 52, 62, 72, 82, 92) traversant le matériau composite et dont le diamètre correspond au moins au diamètre de l'orifice de sortie (111) d'un canal de trayon (110).

12. Film selon l'une des revendications précédentes, **caractérisé en ce qu'**est prévue une couverture (103) permettant de recouvrir de l'extérieur le trou (92) entre les traites.

13. Utilisation d'un film conforme à l'une des revendications précédentes 5 à 12 en vue d'adapter le diamètre d'un trayon à un gobelet trayeur.
